# EUROPEAN PATENT SPECIFICATION

(11) **EP 2 656 846 B1**
(45) Date of publication and mention of the grant of the patent: **30.03.2016**
(21) Application number: 11851679.8
(22) Date of filing: 19.12.2011
(51) Int. Cl.: A61K 31/665, A61K 47/18, A61K 9/14, A61P 31/04, A61P 13/02, A61K 31/655

(54) **PHOSPHOMYCIN PHARMACEUTICAL COMPOSITION**
PHARMAZEUTISCHE PHOSPHOMYCINZUSAMMENSETZUNG
COMPOSITION PHARMACEUTIQUE DE FOSFOMYCINE

(30) Priority: 24.12.2010 ES 201001613
(43) Date of publication of application: 30.10.2013
(73) Proprietor: Arafarma Group, S.A., Marchamalo 19180, Guadalajara (ES)
(72) Inventor: PICORNELL DARDER, Carlos, E-28043 Madrid (ES)
(74) Representative: ZBM Patents - Zea, Barlocci & Markvardsen
(86) International application number: PCT/ES2011/000366
(87) International publication number: WO 2012/085304

(56) References cited:
- EP-A1- 1 747 781
- ES-B1- 2 103 682
- ES-B1- 2 224 869
- ES-B1- 2 244 333
- US-A- 4 727 065
- US-A- 5 162 309
- US-A1- 2004 022 866

## Description

### TECHNICAL FIELD

The present invention relates to a stable pharmaceutical composition for oral administration of the antibiotic fosfomycin.

### BACKGROUND ART

Fosfomycin trometamol, which chemical name is mono(2-ammonium-2-hydroxymethyl-1,3-propanediol) (2R,cis)-(3-methyloxiranyl)phosphonate, is an antibiotic indicated in the prophylaxis and treatment of uncomplicated acute infections of the lower urinary tract.

There are some fosfomycin trometamol pharmaceutical compositions for oral administration disclosed in the state of the art.

The Spanish patent application ES495870 (Zambon, S.p.a.) describes a composition for oral administration in form of sachets containing fosfomycin trometamol, sodium carboxymethylcellulose, lactose, titanium dioxide, orange flavour and sucrose. The sucrose content in this composition, depending on the active substance dosage, changes between 56 and 76 % expressed by weight over the composition total weight.

Another Spanish patent application, ES2020790 (Zambon Group, S.p.a.), discloses a granulated containing fosfomycin trometamol, saccharine, flavour and sucrose. The sucrose content in this composition comprises 28 % expressed by weight over the composition total weight.

The Spanish patent application ES2224869 (Zambon Group, S.p.a.) discloses the use of certain basic compounds to stabilize a fosfomycin trometamol pharmaceutical composition. Some compositions described in the examples also mention the use of between 16 % and 22 % of sucrose expressed by weight over the composition total weight.

The Spanish patent application ES2244333 (Simbec Ibérica, S.L.) discloses a pharmaceutical preparation comprising fosfomycin trometamol, mannitol and/or xylitol and an artificial sweetener selected from the group including acesulfame, aspartame, saccharin, alitame and/or cyclamate. The mannitol and/or xylitol content comprises between 20-40 % expressed by weight over the composition total weight.

The presence of certain sugars such as sucrose, fructose or glucose in a fosfomycin trometamol pharmaceutical composition can arise in an undesirable colouration due to the reaction between the aldehyde groups of such sugars with the trometamol amine or any other primary amino group present in the composition. Furthermore, the presence of these sugars makes the obtained pharmaceutical composition not suitable for the administration to diabetic patients.

On the other hand, it is well known in the state of the art that pharmaceutical compositions comprising a sugar derivative such as a sugar alcohol, in particular xylitol, mannitol, sorbitol or mixtures thereof, can cause intestinal problems such as an undesirable laxative effect or abdominals gases.

### DETAILED DESCRIPTION OF THE INVENTION

The present invention relates to a stable solid fosfomycin pharmaceutical composition for oral administration characterised by comprising fosfomycin trometamol, glycine, optionally other pharmaceutically acceptable excipients, and it is substantially free from sugars and sugar alcohols such as sucrose, fructose, glucose, xylitol, mannitol, sorbitol or mixtures thereof.

By the term "substantially free" it is understood that the sugar or sugar alcohol content is non-existent or so low that fosfomycin pharmaceutical composition of the invention still is stable from colouration, suitable for diabetics and no cause intestinal problems.

Glycine is a neutral amino acid with a pH value between 5.9 and 6.4 according to the European Pharmacopoeia 6.0. Glycine does not have any lateral chain; it is the unique amino acid without chiral centre.

This amino acid has a slight sweet taste that, different from other amino acids with bitter or acid taste, makes it suitable to be used as a diluent in an oral administration pharmaceutical composition. Furthermore, the absence of aldehyde groups on its structure avoids the coloration due to the Maillard reaction with amines making possible to obtain stable from coloration compositions. At the same time, a pharmaceutical composition comprising glycine does not represent any risk for diabetic patients because does not give any glycemic response, and no gastrointestinal problems such as an undesirable laxative effect or abdominal gases are arisen.

The amount of glycine can be easily determined by a person skilled in the art in order to get a fosfomycin trometamol solid oral pharmaceutical composition by known techniques usual in the state of the art. Generally, the amount of glycine presents in the fosfomycin pharmaceutical composition can comprise between 0.1% and 70 % expressed by weight over the composition total weight. Preferably, the amount of glycine comprises between 20 and 50 % by weight over the composition total weight.

Optionally, this fosfomycin trometamol pharmaceutical composition comprises one or more sweeteners to improve its palatability. Preferably, the sweetener is selected from the group including sucralose, acesulfame, aspartame, saccharine, alitame, cyclamate, neohesperidine dihydrochalcone, thaumatina and mixtures thereof.

More preferably, the sweetener used to improve the palatability of the pharmaceutical composition comprising fosfomycin trometamol and glycine is sucralose. This compound is between 300 and 1000 times sweeter than sucrose, does not have the aftertaste characteristic of other artificial sweeteners, is not absorbed by the organism and does not present toxicity problems.

The addition of sucralose to the pharmaceutical composition allows obtain a more pleasant taste using only a small quantity of such a sweetener. The fosfomycin trometamol and glycine pharmaceutical composition of this invention can comprise between 0.0 and 5.0 % of sucralose, preferably between 1.0 and 2.0 % expressed by weight over the composition total weight.

In a preferred embodiment, the fosfomycin pharmaceutical composition is presented in form of powder or granulates for solution. More preferably the powder or granulates for solution are contained in single dosage sachets for solution.

Optionally, the fosfomycin pharmaceutical composition further comprises one or more of pharmaceutically acceptable excipients usual in the pharmaceutical industry to obtain oral administration solid compositions, in particular to obtain pharmaceutical compositions in form of powder or granulate for solution.

Preferably these excipients are selected from the group including glidants such as calcium silicate, magnesium silicate, silicon dioxide, magnesium stearate, magnesium oxide, talc, cellulose, starch or mixtures thereof; binders such as microcrystalline cellulose, pregelatinized starch, copovidone, polyvinylpyrrolidone (povidone), hydroxypropylmethyl cellulose, hydroxypropyl cellulose, hydrogenated vegetable oil or mixtures thereof; and/or flavours such as orange, tangerine, lemon, raspberry, strawberry or coconut.

More preferably, the pharmaceutical composition of the invention further comprises silicon dioxide as glidant, polyvinylpyrrolidone as binder and lemon flavour.

The pharmaceutical composition of this invention generally contains between 1.9 and 7.5 g of fosfomycin trometamol, corresponding to 1.0 and 4.0 g of fosfomycin expressed as free acid. Preferably, the composition contains between 3.8 g and 5.6 g of fosfomycin trometamol, corresponding to 2.0 and 3.0 g of fosfomycin expressed as free acid.

The amount of the active ingredient previously mentioned corresponds to between 30 % and 99.9 % by weight over the composition total weight. Preferably, the content of fosfomycin trometamol represents between 50 % and 80 % by weight over the composition total weight.

The fosfomycin stable solid pharmaceutical composition for oral administration of this invention can be used for the manufacture of a medicament for the treatment or profilaxis of uncomplicated acute infections of the lower urinary tract.

Furthermore, the fosfomycin stable solid pharmaceutical composition for oral administration of this invention can be used for the treatment or profilaxis of uncomplicated acute infections of the lower urinary tract.

The fosfomycin pharmaceutical composition to this invention can be obtained by methods widely known by the person skilled in the art, in particular by mixing and/or granulation methods.

### EXAMPLE: Powder or granulate for solution

The components are homogeneously mixed and, optionally, granulated by known techniques usual in the state of the art. The solid obtained is dosed in sachets containing the quantity equivalent to 2.0 g or 3.0 g of fosfomycin in acid form.

| | |
|---|---|
| Fosfomycin trometamol | 63 % |
| Glycine | 32 % |
| Sucralose | 1.3 % |
| Lemon flavour | 1.4 % |
| Silicon dioxide | 0.1 % |
| Polyvinylpyrrolidone | 2.2 % |
| Total | 100 % |

## Claims

1. Stable solid fosfomycin pharmaceutical composition for oral administration **characterized by** comprising
a) fosfomycin trometamol,
b) glycine and
c) optionally other pharmaceutically acceptable excipients,
and wherein the content of sugars and sugar alcohols selected from sucrose, fructose, glucose, xylitol, mannitol, sorbitol and mixtures thereof in the composition is non-existent or so low that the fosfomycin pharmaceutical composition is still stable from colouration, suitable for diabetics and does not cause intestinal problems.

2. Pharmaceutical composition according to claim 1, **characterized by** further comprising at least a pharmaceutically acceptable sweetener.

3. Pharmaceutical composition according to claim 2, **characterized in that** the sweetener is selected from the group consisting of sucralose, acesulfame, aspartame, saccharine, alitame, cyclamate, neohesperidine dihydrochalcone, thaumatina and mixture thereof.

4. Pharmaceutical composition according to claim 3, **characterised in that** the sweetener is sucralose.

5. Pharmaceutical composition according to any of the previous claims, **characterised by** comprising other pharmaceutically acceptable excipients such as glidants, binders, flavours or mixtures thereof.

6. Pharmaceutical composition according to any of the previous claims, **characterised by** comprising, expressed by weight over the composition total weight:
a) between 30 % and 99.9 % fosfomycin trometamol,
b) between 0.1 and 70 % glycine, and
c) optionally other pharmaceutically acceptable excipients in quantity enough to complete the 100 % of the composition total weight.

7. Pharmaceutical composition according to claim 6, **characterised by** comprising, expressed by weight over the composition total weight:
a) about 63 % fosfomycin trometamol,
b) about 32 % glycine, and
c) optionally other pharmaceutically acceptable excipients in quantity enough to complete the 100 % of the composition total weight.

8. Pharmaceutical composition according to claim 6 or 7, **characterised by** comprising, expressed by weight over the composition total weight:
a) between 30 % and 99.9 % fosfomycin trometamol,
b) between 0.1 and 70 % glycine,
c) between 0.0 and 5.0 % sweetener, and
d) optionally other pharmaceutically acceptable excipients in quantity enough to complete the 100 % of the composition total weight.

9. Pharmaceutical composition according to claim 8, **characterised by** comprising, expressed by weight over the composition total weight:
a) about 63 % fosfomycin trometamol,
b) about 32 % glycine,
c) about 1.3 % sucralose,
d) about 0.1 % silicon dioxide,
e) about 2.2 % polyvinylpyrrolidone, and
f) about 1.4 % lemon flavour.

10. Pharmaceutical composition according to any of the previous claims, **characterised in** being powder or granulate for solution.

11. Pharmaceutical composition according to claim 10, **characterised in that** the powder or granulate for solution is soluble in water.

12. The fosfomycin stable solid pharmaceutical composition as defined in any of the previous claims, for use in the prophylaxis and/or treatment of uncomplicated acute infections of the lower urinary tract.

## Patentansprüche

1. Stabile feste pharmazeutische Fosfomycinzusammensetzung zur oralen Verabreichung, **dadurch gekennzeichnet, dass** sie folgendes umfasst
a) Fosfomycintrometamol,
b) Glycin und
c) wahlweise weitere pharmazeutisch akzeptable Hilfsstoffe,
und wobei der Gehalt an Zuckern und Zuckeralkoholen in der Zusammensetzung, die aus Saccharose, Fructose, Glucose, Xylitol, Mannitol, Sorbitol und Mischungen davon ausgewählt sind nichtexistierend oder so gering ist, dass die pharmazeutische Fosfomycinzusammensetzung noch farbecht ist, geeignet für Diabetiker ist und keine Darmbeschwerden verursacht.

2. Pharmazeutische Zusammensetzung nach Anspruch 1, **dadurch gekennzeichnet, dass** sie weitere mindestens einen pharmazeutisch akzeptablen Süßstoff umfasst.

3. Pharmazeutische Zusammensetzung nach Anspruch 2, **dadurch gekennzeichnet, dass** der Süßstoff ausgewählt aus der Gruppe bestehend aus Sucralose, Acesulfam, Aspartam, Saccharin, Alitam, Cyclamat, Neohesperidin-Dihydrochalkon, Thaumatin und Mischungen davon ist.

4. Pharmazeutische Zusammensetzung nach Anspruch 3, **dadurch gekennzeichnet, dass** der Süßstoff Sucralose ist.

5. Pharmazeutische Zusammensetzung nach einem der vorhergehenden Ansprüche, **dadurch gekennzeichnet, dass** sie andere pharmazeutisch akzeptable Hilfsstoffe wie Fließregulierungsmitte, Bindemittel, Geschmacksstoffe oder Mischungen davon umfasst.

6. Pharmazeutische Zusammensetzung nach einem der vorhergehenden Ansprüche, **dadurch gekennzeichnet, dass** sie, angegeben als Gewichtsverhältnis bezüglich des Gesamtgewichts der Zusammensetzung, folgendes umfasst:
a) zwischen 30 % und 99,9 % Fosfomycintrometamol,
b) zwischen 0,1 und 70 % Glycin, und
c) wahlweise andere pharmazeutisch akzeptable Hilfsstoffe in ausreichender Menge bis 100 % des Gesamtgewichts der Zusammensetzung.

7. Pharmazeutische Zusammensetzung nach Anspruch 6, **dadurch gekennzeichnet, dass** sie, angegeben als Gewichtsverhältnis bezüglich des Gesamtgewichts der Zusammensetzung, folgendes umfasst:
a) ca. 63 % Fosfomycintrometamol,
b) ca. 32 % Glycin, und
c) wahlweise andere pharmazeutisch akzeptable Hilfsstoffe in ausreichender Menge bis 100 % des Gesamtgewichts der Zusammensetzung.

8. Pharmazeutische Zusammensetzung nach Anspruch 6 oder 7, **dadurch gekennzeichnet, dass** sie, angegeben als Gewichtsverhältnis bezüglich des Gesamtgewichts der Zusammensetzung, folgendes umfasst:
a) zwischen 30 % und 99,9 % Fosfomycintrometamol,
b) zwischen 0,1 und 70 % Glycin,
c) zwischen 0,0 und 5,0 % Süßstoff, und
d) wahlweise andere pharmazeutisch akzeptable Hilfsstoffe in ausreichender Menge bis 100 % des Gesamtgewichts der Zusammensetzung.

9. Pharmazeutische Zusammensetzung nach Anspruch 8, **dadurch gekennzeichnet, dass** sie, angegeben als Gewichtsverhältnis bezüglich des Gesamtgewichts der Zusammensetzung, folgendes umfasst:
a) ca. 63 % Fosfomycintrometamol,
b) ca. 32 % Glycin,
c) ca. 1,3 % Sucralose,
d) ca. 0,1 % Siliziumdioxid,
e) ca. 2,2 % Polyvinylpyrrolidon, und
f) ca. 1,4 % Zitronengeschmacksstoff.

10. Pharmazeutische Zusammensetzung nach einem der vorhergehenden Ansprüche, **dadurch gekennzeichnet, dass** sie ein Pulver oder ein Granulat zur Lösung ist.

11. Pharmazeutische Zusammensetzung nach Anspruch 10, **dadurch gekennzeichnet, dass** das Pulver bzw. das Granulat zur Lösung in Wasser lösbar sind.

12. Die stabile feste pharmazeutische Fosfomycinzusammensetzung wie in einem der vorhergehenden Ansprüche definiert zur Verwendung bei der Prophylaxe und/oder der Behandlung von unkomplizierten akuten Infektionen des unteren Harntraktes.

## Revendications

1. Composition pharmaceutique de fosfomycine solide stable pour l'administration orale **caractérisée en ce qu'**elle comprend
a) fosfomycine trométamol,
b) glycine et
c) facultativement d'autres excipients pharmaceutiquement acceptables,
et dans laquelle la teneur en sucres et alcools de sucre choisis parmi le saccharose, le fructose, le glucose, le xylitol, le mannitol, le sorbitol et des mélanges de ceux-ci dans la composition est non-existant ou si faible que la composition pharmaceutique de fosfomycine est toujours stable en termes de décoloration, appropriée pour des personnes diabétiques et elle n'entraîne pas des problèmes intestinaux.

2. Composition pharmaceutique selon la revendication 1, **caractérisée en ce qu'**elle comprend en outre au moins un édulcorant pharmaceutiquement acceptable.

3. Composition pharmaceutique selon la revendication 2, **caractérisée en ce que** l'édulcorant est choisi dans le groupe constitué du sucralose, l'acésulfame, l'aspartame, la saccharine, l'alitame, le cyclamate, la néohespéridine dihydrochalcone, la thaumatine et des mélanges de ceux-ci.

4. Composition pharmaceutique selon la revendication 3, **caractérisée en ce que** l'édulcorant est la sucralose.

5. Composition pharmaceutique selon l'une quelconque des revendications précédentes, **caractérisée en ce qu'**elle comprend d'autres excipients pharmaceutiquement acceptables tels que des agents de glissement, des liants, des arômes ou des mélanges de ceux-ci.

6. Composition pharmaceutique selon l'une quelconque des revendications précédentes, **caractérisée en ce qu'**elle comprend, exprimé par rapport au poids total de la composition :
a) entre 30 % et 99,9 % fosfomycine trométamol,
b) entre 0,1 et 70 % glycine, et
c) facultativement d'autres excipients pharmaceutiquement acceptables dans une quantité suffisante pour compléter le 100 % du poids total de la composition.

7. Composition pharmaceutique selon la revendication 6, **caractérisée en ce qu'**elle comprend, exprimé par rapport au poids total de la composition :
a) environ 63 % fosfomycine trométamol,
b) environ 32 % glycine, et
c) facultativement d'autres excipients pharmaceutiquement acceptables dans une quantité suffisante pour compléter le 100 % du poids total de la composition.

8. Composition pharmaceutique selon la revendication 6 ou 7, **caractérisée en ce qu'**elle comprend, exprimé par rapport au poids total de la composition :
a) entre 30 % et 99,9 % fosfomycine trométamol,
b) entre 0,1 et 70 % glycine,
c) entre 0,0 et 5,0 % édulcorant, et
d) facultativement d'autres excipients pharmaceutiquement acceptables dans une quantité suffisante pour compléter le 100 % du poids total de la composition.

9. Composition pharmaceutique selon la revendication 8, **caractérisée en ce qu'**elle comprend, exprimé par rapport au poids total de la composition :
a) environ 63 % fosfomycine trométamol,
b) environ 32 % glycine,
c) environ 1,3 % sucralose,
d) environ 0,1 % dioxyde de silicium,
e) environ 2,2 % polyvinylpyrrolidone, et
f) environ 1,4 % arôme de citron.

10. Composition pharmaceutique selon l'une quelconque des revendications précédentes, **caractérisée en ce qu'**elle est sous forme de poudre ou de granulés pour solution.

11. Composition pharmaceutique selon la revendication 10, **caractérisée en ce que** la poudre ou les granulés pour solution sont solubles dans l'eau.

12. La composition pharmaceutique solide stable de fosfomycine telle que définie dans l'une quelconque des revendications précédentes, pour l'utilisation dans la prophylaxie et/ou le traitement d'infections aiguës non compliquées des voies urinaires inférieures.
